# EUROPEAN PATENT APPLICATION

(11) **EP 1 902 715 A2**
(43) Date of publication of application: **26.03.2008**
(21) Application number: 07253744.2
(22) Date of filing: 21.09.2007
(51) Int. Cl.: A61K 31/517, A61P 1/16, A61P 25/18, A61P 25/22, A61P 25/24, A61P 25/32

(54) **Use of paliperidone for the treatment of psychiatric patients with reduced hepatic function**

(30) Priority: 22.09.2006 US 534623
(71) Applicant: Janssen Pharmaceutica, N.V., 2340 Beerse (BE)
(72) Inventor: Boom, Sandra, 3317 BT Dordrecht (NL); Erdekens, Marielle-Henriette, 3650 Dilsen-Stokkem (BE)
(74) Representative: Warner, James Alexander

(57) **Abstract**

The present invention provides a method for the treatment of psychiatric patients having or at risk of hepatic impairment comprising administering a therapeutically effective amount of paliperidone, its pharmaceutically acceptable acid addition salts, enantiomeric forms and esters thereof to psychiatric patients in need thereof.

## Description

### FIELD OF THE INVENTION

This invention relates to the treatment of patients that are hepatically impaired with paliperidone.

### BACKGROUND OF THE INVENTION

Psychiatric patients often have comorbid conditions that may lead to hepatic impairment. Consequently, to treat these patients with comorbid conditions that potentially cause impaired hepatic function it would be highly desirable to be able to treat these patients for their mental illness with pharmaceuticals that are not to any appreciable degree metabolized in the liver.

We have discovered that paliperidone, its pharmaceutically acceptable acid addition salts, enantiomeric forms and esters are not to any appreciable extent metabolized in the liver therefore, are particularly useful in the treatment of psychiatric patients with impaired liver function.

### SUMMARY OF THE INVENTION

In one embodiment the present invention provides a method for the treatment of psychiatric patients having or at risk of hepatic impairment comprising administering a therapeutically effective amount of paliperidone, its pharmaceutically acceptable acid addition salts, enantiomeric forms and esters thereof to psychiatric patients in need thereof.

This and other objects and advantages of the present invention may be appreciated from a review of the present applications.

### DETAILED DESCRIPTION

A variety of comorbid conditions which psychiatric patients may present also may lead to hepatic impairment in psychiatric patients such as Wilson's disease, alcoholism, viral hepatitis (e.g. hepatitis B, Hepatitis C adenovirus, Epstein Barr virus, cytomegalovirus and viral haemorraghic fevers), drug toxicity, hepatocellular carcinoma or metastatic carcinoma, poisoning by various substances, illicit drug use (including Ecstacy and cocaine), Reyes' syndrome, Budd-Chiari syndrome, veno-occlusive disease and autoimmune liver disease. Psychiatric patients that are suspected of hepatic impairment can be identified by examination of their medical records, taking their histories, physical examination or by laboratory testing. Physicians and nurses treating psychiatric patients should be familiar with the symptoms and tests for impaired liver functions. For example patients presenting with symptoms such as jaundice, liver palms, cerebral oedema, etc. should be further examined for liver impairment. Laboratory tests showing thrombocytopenia, raised bilirubin, low pseudocholinesterase, elevated lactate, raised creatinine etc., should be further investigated. Appropriate techniques to determine whether there is impairment of liver function is know in the art. Normally a battery of tests will be run such as test for the levels of transaminase (e.g. aspartate aminotransferase, alanine aminotransferase, etc.) and γ-glutamyltransferase, Hepatitis C serologies, Hepatitis B serologies Hepatitis A serology, Ceruloplasmin, serum protein electrophoresis, hepatic sonogram prothrombin time, CBC with platelet count and serum albumin.

Hepatically impaired patients are those patients that one of ordinary skill in the art such as a physician would recognize from testing or diagnosis that had impaired liver function requiring monitoring of their liver condition and/or care being taken in the administration of medication to avoid adverse events (e.g. further damage or failure to properly metabolize and/or clear medicines).
Paliperidone including its pharmaceutically acceptable acid addition salts, enantiomeric forms and esters may be administered for the practice of the present invention. Paliperidone is described in US Patent 5,5158,952 incorporated herein by reference. Esters of paliperidone are described in US Patent 5,254,556 incorporated herein by reference. Esters of paliperidone include octanoic acid, decanoic acid, dodecanic acid, tetradecanoic acid or hexadecanoic acid (palmitic acid). The currently preferred ester of paliperidone is paliperidone palmitate.

Paliperidone may be formulated with pharmaceutical excipients into a variety of dosage forms as described in US Patent 5,158,952. Paliperidone will in one embodiment of the present invention be provided in an oral dosage forms. Suitable oral dosage forms include but are not limited to tablets, pills, fast dissolving dosage forms, controlled release or extended release dosage forms. Currently preferred are extended release OROS oral dosage forms such as those described in US 2004092534, US 200508132 and US 2005232995 hereby incorporated herein by reference.

Paliperidone palmitate may also be formulated with pharmaceutical excipients into a variety of dosage forms as described in US Patent 5,254,556. Currently it is preferred to administer paliperidone palmitate in a depot. Suitable aqueous depot formulations are described in US Patent 6,077,843, US Patent 6,320,048 and US Patent 6,555,544.

The term "psychiatric patient" as used herein, refers to a human, who has been the object of treatment, or experiment for a "mental disorder" and "mental illness" refer to those provided in the Diagnostic and Statistical Manual (DSM IV), American Psychological Association (APA). Those of ordinary skill in the art will appreciate that paliperidone, its salts, enatiomers and esters can be administered to psychiatric patients for all the known uses of risperidone. These mental disorders include, but are not limited to, schizophrenia; bipolar disorder or other disease states in which psychosis, aggressive behavior, anxiety or depression is evidenced. Schizophrenia refers to conditions characterized as schizophrenia, schizoaffective disorder and schizophreniform disorders, in DSM-IV-TR such as category 295.xx. Bipolar Disorder refers to a conditions characterized as a Bipolar Disorder, in DSM-IV-TR such as category 296.xx including Bipolar I and Bipolar Disorder II. The DSM-IV-TR was prepared by the Task Force on Nomenclature and Statistics of the American Psychiatric Association, and provides clear descriptions of diagnostic categories. Pathologic psychological conditions, which are psychoses or may be associated with psychotic features include, but are not limited to the following disorders that have been characterized in the DSM-IV-TR. Diagnostic and Statistical Manual of Mental Disorders, Revised, 3rd Ed. (1994). The numbers in parenthesis refer to the DSM-IV-TR categories. The skilled artisan will recognize that there are alternative nomenclatures, nosologies, and classification systems for pathologic psychological conditions and that these systems evolve with medical scientific progress.
Examples of pathologic psychological conditions which may be treated include, but are not limited to, Mild Mental Retardation (317), Moderate Mental Retardation (318.0), Severe Mental Retardation (318.1), Profound Mental Retardation (318.2), Mental Retardation Severity Unspecified (319), Autistic Disorders (299.00), Rett's Disorder (299.80), Childhood Disintegrative Disorders (299.10), Asperger's Disorder (299.80), Pervasive Developmental Disorder Not Otherwise Specified (299.80), Attention-Deficit/Hyperactivity Disorder Combined Type (314.01), Attention-Deficit/Hyperactivity Disorder Predominately Inattentive Type (314.00), Attention-Deficit/Hyperactivity Disorder Predominately Hyperactive-Impulsive Type (314.01), Attention-Deficit/Hyperactivity Disorder NOS (314.9) Conduct Disorder (Childhood-Onset and Adolescent Type 312.8) Oppositional Defiant Disorder (313.81), Disruptive Behavior Disorder Not Otherwise Specified (312.9), Solitary Aggressive Type (312.00), Conduct Disorder, Undifferentiated Type (312.90), Tourette's Disorder (307.23), Chronic Motor Or Vocal Tic Disorder (307.22), Transient Tic Disorder (307.21), Tic Disorder NOS (307.20), Alcohol Intoxication Delirium (291.0), Alcohol Withdrawal Delirium (291.0), Alcohol-Induced Persisting Dementia (291.2), Alcohol-Induced Psychotic Disorder with Delusions (291.5), Alcohol-Induced Psychotic Disorder with Hallucinations (291.3), Amphetamine or Similarly Acting Sympathomimetic Intoxication (292.89), Amphetamine or Similarly Acting Sympathomimetic Delirium (292.81), Amphetamine or Similarly Acting Sympathomimetic Induced Psychotic with Delusional (292.11), Amphetamine or Similarly Acting Sympathomimetic Induced Psychotic with Hallucinations (292.12), Cannabis-Induced Psychotic Disorder with Delusions (292.11), Cannabis-Induced Psychotic Disorder with Hallucinations (292.12), Cocaine Intoxication (292.89), Cocaine Intoxication Delirium (292.81), Cocaine-Induced Psychotic Disorder with Delusions (292.11), Cocaine-Induced Psychotic Disorder with Hallucinations (292.12), Halluciogen Intoxication (292.89), Hallucinogen Intoxication Delirium (292.81), Hallucinogen-Induced Psychotic disorder with Delusions (292.11), Hallucinogen-Induced Psychotic disorder with Delusions (292.12), Hallucinogen-Induced Mood Disorder (292.84), Hallucinogen-Induced Anxiety Disorder (292.89), Hallucinogen-Related Disorder Not Otherwise Specified (292.9), Inhalant Intoxication (292.89), Inhalant Intoxication Delirium (292.81), Inhalant-Induced Persisting Dementia (292.82), Inhalant-Induced Psychotic Disorder with Delusions (292.11), Inhalant-Induced Psychotic with Hallucinations (292.12), Inhalant-Induced Mood Disorder (292.89), Inhalant-Induced Anxiety Disorder (292.89), Inhalant-Related Disorder Not Otherwise Specified (292.9), Opioid Intoxication Delirium (292.81), Opioid-Induced Psychotic Disorder with Delusions (292.11), Opioid Intoxication Delirium (292.81), Opioid-Induced Psychotic Disorder with Hallucinations (292.12), Opioid-Induced Mood Disorder (292.84), Phencyclidine (PCP) or Similarly Acting Arylcyclohexylamine Intoxication (292.89), Phencyclidine (PCP) or Similarly Acting Arylcyclohexylamine Intoxication Delirium (292.81), Phencyclidine (PCP) or Similarly Acting Arylcyclohexylamine Induced Psychotic Disorder with Delusions (292.11), Phencyclidine (PCP) or Similarly Acting Arylcyclohexylamine Induced Psychotic Disorder with Hallucinations (292.12), Phencyclidine (PCP) or Similarly Acting Arylcyclohexylamine Mood Disorder (292.84), Phencyclidine (PCP) or Similarly Acting Arylcyclohexylamine Induced Anxiety Disorder (292.89), Phencyclidine (PCP) or Similarly Acting Arylcyclohexylamine Related Disorder Not Otherwise Specified (292.9), Sedative, Hypnotic or Anxiolytic Intoxication (292.89), Sedation, Hypnotic or Anxiolytic Intoxication Delirium (292.81), Sedation, Hypnotic or Anxiolytic Withdrawal Delirium (292.81), Sedation, Hypnotic or Anxiolytic Induced Persisting Dementia (292.82), Sedation, Hypnotic or Anxiolytic-Induced Psychotic Disorder with Delusions (292.11), Sedation, Hypnotic or Anxiolytic-Induced Psychotic Disorder with Hallucinations (292.12), Sedation, Hypnotic or Anxiolytic-Induced Mood Disorder (292.84), Sedation, Hypnotic or Anxiolytic-Induced Anxiety Disorder (292.89), Other (or Unknown) Substance Intoxication (292.89), Other (or Unknown) Substance-Induced Delirium (292.81), Other (or Unknown) Substance-Induced Persisting Dementia (292.82), Other (or Unknown) Substance-Induced Psychotic Disorder with Delusions (292.11), Other (or Unknown) Substance-Induced Psychotic Disorder with Hallucinations (292.12), Other (or Unknown) Substance-Induced Mood Disorder (292.84), Other (or Unknown) Substance-Induced Anxiety Disorder (292.89), Other (or Unknown) Substance Disorder Not Otherwise Specified (292.9), Obsessive Compulsive Disorder (300.3), Post-traumatic Stress Disorder (309.81), Generalized Anxiety Disorder (300.02), Anxiety Disorder Not Otherwise Specified (300.00), Body Dysmorphic Disorder (300.7), Hypochondriasis (or Hypochondriacal Neurosis) (300.7), Somatization Disorder (300.81), Undifferentiated Somatoform Disorder (300.81), Somatoform Disorder Not Otherwise Specified (300.81), Intermittent Explosive Disorder (312.34), Kleptomania (312.32), Pathological Gambling (312.31), Pyromania (312.33), Trichotillomania (312.39), and Impulse Control Disorder NOS (312.30), Schizophrenia, Paranoid Type, (295.30), Schizophrenia, Disorganized (295.10), Schizophrenia, Catatonic Type, (295.20), Schizophrenia, Undifferentiated Type (295.90), Schizophrenia, Residual Type (295.60), Schizophreniform Disorder (295.40), Schizoaffective Disorder (295.70), Delusional Disorder (297.1), Brief Psychotic Disorder (298.8), Shared Psychotic Disorder (297.3), Psychotic Disorder Due to a General Medical Condition with Delusions (293.81), Psychotic Disorder Due to a General Medical Condition with Hallucinations (293.82), Psychotic Disorders Not Otherwise Specified (298.9), Major Depression, Single Episode, Severe, without Psychotic Features (296.23), Major Depression, Recurrent, Severe, without Psychotic Features (296.33), Bipolar Disorder, Mixed, Severe, without Psychotic Features (296.63), Bipolar Disorder, Mixed, Severe, with Psychotic Features (296.64), Bipolar Disorder, Manic, Severe, without Psychotic Features (296.43), Bipolar Disorder, Manic, Severe, with Psychotic Features (296.44), Bipolar Disorder, Depressed, Severe, without Psychotic Features (296.53), Bipolar Disorder, Depressed, Severe, with Psychotic Features (296.54), Bipolar 11 Disorder (296.89), Bipolar Disorder Not Otherwise Specified (296.80), Personality Disorders, Paranoid (301.0), Personality Disorders, Schizoid (301.20), Personality Disorders, Schizotypal (301.22), Personality Disorders, Antisocial (301.7), and Personality Disorders, Borderline (301.83).

The term "therapeutically effective amount" as used herein, means that amount of active compound or pharmaceutical agent that elicits the biological or medicinal response in human that is being sought by a researcher, medical doctor or other clinician, which includes alleviation of the symptoms of the disease or disorder being treated.

Those of skill in the treatment of diseases could easily determine the effective amount of paliperidone to administer for the treatment of the diseases listed above. In general it is contemplated that an effective amount would be from about 0.01 mg/kg to about 2 mg/kg body weight. In one embodiment of present invention wherein paliperidone is orally administered a dosage form to a subject once a day is preferred. The mg of compound delivered in such a dosage form to the patient may be from 0.25 to about 20 mg (e.g. 0.25 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, and 20 mg) per oral dosage form.

The following non-limiting examples are provided to further illustrate the present invention.

### EXAMPLE 1

**Objectives:** to investigate the metabolic pathways of paliperidone and excretion of paliperidone and its metabolites in healthy adult make subjects, both CYP2D6 poor and extensive metabolizers, after administration of a single 1-mg oral dose of ¹⁴C-pallperidone. In addition, to evaluate the safety and tolerability of paliperidone, as well as the relationship between genotypes (CYP2D6, CYP3A4, CYP3A5, UGT1A1, and UGT1A6) and exposure to paliperidone and its metabolities.

**Methodology:** Single-center, single-dose open-label study of the absorption, metabolism, and excretion (AME) of paliperidone in healthy men (3 extensive and 3 poor metablizers based on CPY2D6 phenotype). Eligible subjects were admitted to the study center the evening before study drug administration and remained at the study center until 168 hours after dosing (or longer if required up to a maximum of 14 days). Each subject received a single oral dose of ¹⁴C-paliperidone with total radioactivity below 10000 µSv (16 µCi). Blood samples for plasma pharmacokinetic profile were obtained immediately before study drug administration and 0.5, 1, 1.5, 3, 6, 12, 16, 36, 48, 72, 96, 120, 144 and 168 hours postdose. Blood samples were obtained 2, 4, 8, and 24 hours postdose for determination of ¹⁴C in whole blood. Samples for determination of serum cretinine were obtained 2, 4, 8, and 24 hours postdose. Urine was colleted immediately prior to drug administration and from 0-4, 4-8, 8-12, 12-16, 16-24, 24-36, 36-48, 48-72, 96-120, 120-144, and 144-168 hours after study drug administration. Fecal samples were colleted per each stool, once before study drug administration and in the period from 0-168 hours after study drug administration. Collections of urine and feces (per 24 hours) were to continue beyond 168 hours, to a maximum of 336 hours (Day 15) for subjects who excrete radioactivity slowly (2 latest 24-hour urine collections each ≥2% of total radioactive dose) or have <7 feces stool samples over the 0 to 168-hour period. ¹⁴C radioactivity was measured in plasma, urine, and feces. Aliquots of the 0- through 24-hour urine collections were analyzed for cretinine. Plasma concentrations of paliperidone and risperidone were determined by means of a validated LC-MS/MS method. The ¹⁴C-labeled moiety in plasma and urine was determined by liquid scintillation counting. For all plasma samples, the lower limits of quantification for paliperidone and risperidone were 0.100 ng/mL. For all plasma and urine samples the lower limits of quantification for ¹⁴C-palieridone was 72 dpm/mL (=2.0n g-eq/mL).

**Number of Subjects (planned and analyzed):** Six healthy men, 3 extensive and 3 poor metabolizes based on CYP2D6 phenotype were to participate in the study. Five subjects, 3 extensive and 2 poor metabolizers, received a single dose of ¹⁴C-paliperidone, completed the study (i.e., completed all assessments through Day 8) and were considered valuable for safety (the Safety Analysis Set), as well as for pharmacokinetics.

**Diagnosis and Main Criteria for Inclusion:** Subjects were healthy white males between the ages of 40 and 60 years. Subjects were healthy based on medical history, physical examination, clinical laboratory evaluate, and electrocardiogram. Dextromethophan metabolic ratio of ≥0.345 (poor metabolizer) or <0.0255 (extensive metabolize). Body Mass Index (MBI): (weight [kg]/height [m²] between 20 and 28 kg/m², inclusive.

**Test Product, Dose and Mode of Administration Batch No.**: Single oral 0.988-mg dose of ¹⁴ C-pallperidone, oral solution (aqueous formulation at a final concentration of 0.0984 mg/mL). Batch No.: unlabeled paliperidone oral solution: ZR076477EIA031 (manufacturing date: 23 April 2003, retest date: 23 October 2004): ¹⁴C-paliperidone: 1763 (expiration date: not applicable)

**Criteria for Evaluation:** Pharmacokinetics: Plasma paliperidone. ¹⁴C radioactivity and metabolite profiles were determined. Plasma Cₘₐₓ, tₘₐₓ, AUCₗₐₛₜ, AUC₂₄, AUC_{∞}, λ_{Z}, t_{½term}, CL/F of ¹⁴C and paliperidone were estimated by non-compartmental analysis. Based on the individual urine excretion data and on the serum creatinine concentrations, Ae, Ae, % dose, CL_{R}, CL_{R,24h} and CLₑᵣ and paliperidone and CL_{GFR}, and CL_{act} of paliperidone were estimated. The excretion half-life of ¹⁴C in urine was also estimated based on excretion rate-time profiles.

Safety: Evaluation was based on the incidence, type, and severity of all treatment-emergent adverse events, and on change from screening to the end of the study in clinical laboratory results, vital sign measurements, and postural changes in blood pressure and heart rate.

**Statistical Methods:** Pharmacokinetics: Plasma concentrations of ¹⁴C and paliperdone as well as estimates for pharmacokinetic parameters were listed and graphically presented, and the excretion analysis of total radioactivity in plasma, urine and feces were summarized. Descriptive statistics were calculated, including summaries by CYP2D6 phenotype.

Safety: The number of subjects with adverse events was summarized. Summary statistics were calculated for clinical laboratory values. Other safety data were listed by individual sect. Changes in blood pressure and heart rate measurements were also presented graphically.

### Summary-Conclusion

Demographic and baseline characteristics: Five white men, 3 extensive metabolizers and 2 poor metabolizers, received study mediation and completed the study. The ages ranged from 40 to 63 years (mean: 51.2 year), the body weights ranged from 68.7 to 78.6 kg (mean. 73.38 kg), and BMI ranged from 24 to 28 kg/m² (mean: 25.5 kg/m²).

### Pharmacokinetic Results:

Pharmacokinetics of Total Radioactivity (TR) and paliperidone (UD) in plasma;

The mean (SD) pharmacokinetic parameters of TR and UD after administration of a single oral dose of ¹⁴C-paliperidone are summarized in Table A.

**Table A: Plasma Pharmacokinetic Parameters of ¹⁴C-Labeled Moiety and Unchanged Paliperidone (Mean ± SD) After a Single Dose of 1-mg ¹⁴C-Paliperidone**

| | | | |
|---|---|---|---|
| | | | |

| | **ALL (N=5)** | **EM(N=3)** | **PM (N=2)** |
|---|---|---|---|
| **¹⁴C-Labeled Moiety (TR)** | | | |
| C_{max,ng-equivalent/mL} | 9.54 ± 1.35 | 9.40 ± 1.73 | 9.75 ± 1.06 |
| **t**ₘₐₓ,h | 1.40 ± 0.224 | 1.50 ± 0.00 | 1.25 ± 0.354 |
| AUC₂₄,ng-eqh/mL | 114 ± 19.9 | 116 ± 27.3 | 112 ± 7.8 |
| AUC₂₄ng-eq h/mL | 175 ± 30.7 | 179 ± 41.9 | 168 ± 9.90 |
| **t**_{1/2term}, h | 15.2 ± 2.15 | 15.4 ± 1.35 | 14.9 ± 3.82 |
| CL/F,mL/min | 97.9 ± 17.6 | 96.8 ± 24.4 | 99.6 ± 6.22 |

| **Unchanged Paliperidone (UD)** | | | |
|---|---|---|---|
| Cₘₐₓ ng/mL | 8.85 ± 1.31 | 8.59 ± 1.79 | 9.24 ± 0.00707 |
| **t**_{max,}h | 1.30 ± 0.274 | 1.33 ± 0.289 | 1.25 ± 0.354 |
| AUC₂₄,ngh/mL | 111 ± 22.0 | 113 ± 30.1 | 109 ± 9.90 |
| AUC_{∞} ng-h/mL | 187 ± 29.3 | 190 ± 38.4 | 182 ± 19.8 |
| **t**_{½term},h | 24.8 ± 4.35 | 24.1 ± 4.49 | 26.0 ± 5.59 |
| CL/F, mL/min | 91.0 ± 15.0 | 90.3 ± 20.0 | 92.1 ± 10.0 |
| Ratio AUG₂₄: UD/TR | 0.970 ± 0.0250 | 0.965 ± 0.0311 | 0.977 ± 0.0205 |
| EM: extensive metabolizer; PM: poor metabolizer | | | |

In the overall population, average peak plasma concentration of TR (9.54 ng-eq/mL) was attained 1.40 hours after dosing. Average peak plasma concentration of UD (8.85 ng/mL) was reached after 1.30 hours. The terminal half-life of TR and UD was on average 15.2 hours and 24.9 hours, respectively, This difference was probably caused by a higher LLOQ for TR compared to UD, AUC_{∞} values of TR averaged 175 ng-eq.h/mL, those of UD were 187 ng.h/mL. At 24 hours after dosing the percentage of UD versus TR in plasma is on average 97.0%. No differences are found between CYP2D6 extensive and poor metabolizers.

### Excretion in Urine and Feces:

At 7 days after a single oral dose of ¹⁴C-pallperione, 91% of the administered radioactivity has been excreted as ¹⁴C-labeled moiety. The cumulative excretion of the TR amounted to 80% in the urine (Table B) and 11% in the feces. There were no differences between extensive and poor metabolizers in urinary excretion (% of the dose) of ¹⁴C-labeled moiety. Furthermore no discrimination could be made between extensive (13%) and poor (8%) metabolizers of excretion of ¹⁴C labeled moiety in feces.

**Table B: Clearance and Urine Parameters of ¹⁴C-Labled Moiety (Mean ± SD) After Single Dose of 1-mg ¹⁴C Paliperidone**

| | **All (N=5)** | **EM (N=3)** | **PM (N=2)** |
|---|---|---|---|
| **¹⁴C-Labled Moiety** | | | |
| Ae, % dose | 79.6 ± 4.20 | 77.6 ± 0.775 | 82.7 ± 6.15 |
| CL_{R}, mL/min | 76.8 ± 13.6 | 74.1 ± 18.5 | 80.8 ± 1.20 |
| CL_{CR}, mL/i-nin | 113 ± 10.3 | 108 ± 7.37 | 121 ± 10.5 |

| Unchanged Paliperidone | | | |
|---|---|---|---|
| Ae, % dose | 59.4 ± 7.12 | 55.7 ± 6.66 | 64.9 ± 3.68 |
| CL_{R}, mL/min | 53.1 ± 9.47 | 49.2 ± 8.59 | 59.1 ± 9.69 |

### Safety Results:

Two of the 5 subjects who received study education experienced treatment-emergent adverse events: moderately severe postural hypotension and syncope in 1 subject and mild allergic reaction (described as infraorbital swelling probably due to an allergic reaction, and considered doubtfully related to the study medication) and asthenia in the second subject. With the exception of allergic reaction which was persisting at the end of the study, the adverse events resolved without treatment intervention. There were no deaths, no serious adverse events, and no subject discontinued from the study due to an adverse event.

There were no clinically relevant changes in laboratory test results. With the exception of postural hypotension which was reported as an adverse event in 1 subject, there were no clinically relevant changes in vital sign measurements.

### Conclusion:

The unchanged drug paliperidone accounted for a large part of the total radioactivity in plasma. The percentage of UD versus TR in plasma is on average 97%. There were no differences in paliperdone pharmacokinetic parameters observed between CYP2D6 extensive and poor metabolizers. No effect of genotype was observed for CYP2D6, UGT1A1 or UGT1A6 on the plasma exposure of TR and UD.

Seven days after administration of a single oral dose of 1 mg ¹⁴C-paliperidone to 5 healthy male subjects, 91% of the dose was excreted in urine and feces as ¹⁴C-labeled moiety. The cumulative excretion of the TR amounted to 80% in the urine and 11% in the feces. The cumulative urinary excretion of unchanged paliperidone amounted to 59%. About 50% of the UD is excreted by means of filtration; the other half of UD is cleared renally by active processes.

The administration of a single oral 1-mg dose of ¹⁴C-paliperidone as a normal solution was safe and well tolerated in healthy men.

### EXAMPLE 2

**Objectives:** The primary objective of this study was to investigate the single-dose pharmacokinetics of immediate-release (IR) paliperidone, after oral administration, in subjects having moderate hepatic impairment ("hepatically-impaired subjects") compared with subjects having normal hepatic function ("healthy subjects"). The secondary objective was to document the plasma protein binding and disposition of the enantiomers of paliperidone. In addition, the tolerability and safety profile of IR paliperidone was compared between hepatically impaired subjects and health subjects.

**Methodology:** This was a single-dose, parallel-group, open-label, single-center, Phase I study of IR paliperidone in subjects having either normal or moderately impaired hepatic function. The groups, consisting of 10 subjects each, were demographically matched with respect to age, weight, sex, and ethnicity. The study considered of a screening period of up to 3 weeks and anope-label, single-dose treatment period (Days 1 through 5). On Day 1, a single dose of 1 mg IR paliperidone oral solution was administered after a fast of at least 10 hours; subjects continued to fast for 4 hours following study drug administration. The 96-hour follow-up consisted of serial sample collection of blood and urine for pharmacokinetic analysis and safety and tolerability assessments. Subjects remained confined to the study site through the 72-hour pharmacokinetics sampling, and consumed standard institutional meals while in the study site. Subjects were released after the 72-hour sampling, then returned to the study site on Day 5 before the 96-hour pharmacokinetics sampling: end-of-study procedure were performed immediately hereafter. A blood sample for DNA isolation was collected to allow for genetic analysis as necessary.

**Number of Subjects (planned and analyzed):** Ten subjects were planned for each hepatic function group; 10 subjects in each group completed the study and were analyzed for pharmacokinetics and safety.

**Diagnosis and Main Criteria for Inclusion:** The study was conducted in men and women, aged 18 through 75 years, inclusive. One group of subjects had moderate hepatic impairment, with stable hepatic disease, a total Child-Pugh score of between 7 and 9, inclusive, and blood pressure that was controlled and stable on antihypertensive agents; the other group had normal hepatic function.

**Test Product, Dose and Mode of Administration, Batch No:** 1 mg IR paliperidone (R076477) oral solution; batch 04C29/F044.

**Duration of Treatment:** This was a single-dose study.

**Criteria for Evolution:** Pharmacokinetics: Plasma and urine concentrations of the paliperidone enantiomers (+) R078543 and (-) R078544 were determined using an LC-MS/MS method. Concentrations of paliperidone were calculated as the sum of the enantiomer concentrations. In addition, serum and urine concentrations of creatinine were determined for the calculation of CL_{CR}. The protein biding and unbound fraction was determined for the 2 paliperidone enantiomers. The unbound fraction for paliperidone was calculated.

Based on the actual pharmacokinetic blood sampling times and actual urine collection periods, the following plasma and urine pharmacokinetic parameters were determined for paliperidone and its enantiomers: Cₘₐₓ, tₘₐₓ, tₗₐₛₜ, AUCₗₐₛₜ, λ_{z}, t_{½}, AUC_{∞}, %AUC_{∞,ex,} CL/F, AUC_{∞} +/- ratio, C,+/- ratio per time point, unbound AUC_{∞}, unbound CL/F or unbound CL (if relevant), Ae (per collection interval and overall), Ae,%dose, Excr. Rate, Vd₂, CR_{R}, CL_{GFR}, CL_{act}, CL_{act}/CL_{R}, CL_{act}/(CUF), CL_{CR}, and CL_{NR}.

Safety: Adverse events, clinical laboratory tests, including prolactin, vital sign measurements, physical examinations, and 12-lad electrocardiograms (ECGs) were analyzed to assess safety.

### Statistical Methods:

Pharmacokinetics: Descriptive statistics were evaluated for the plasma concentrations at each sampling time, and for all pharmacokinetic parameters of paliperidone and its enantiomers for each hepatic function group Graphical exploration of the paliperidone and enantiomer plasma concentrations and urine data, and the derived pharmacokinetic parameters, was performed. In addition, the enantiomer disposition was compared between the groups.

Log-transformed PK parameters were fit to a general linear model with hepatic function grip as fixed effect.

Safety: All subjects were analyzed; statistical analyses were description.

### Summary-Conclusions

Pharmacokinetic Results: The fraction of unbound paliperidone in plasma was higher in hepatically-impaired subjects compared with healthy subjects and averaged 0.353 and 0.279 respectively. The difference in plasma protein binding between the groups most likely results from the reduced α₁-acid glycoprotein (α₁-AGP) plasma concentration in hepatically-impaired subjects, since the fraction of unbound drug appears to be inversely related to the α₁-AGP plasma concentration.

| **Predose Plasma Concentration of Albumin,** α₁-AGP, **and Total Protein and Unbound Fraction for Paliperidone, (+)R078543, and (-)R078544** | | |
|---|---|---|
| | Healthy Subjects (n=10) | Hepatically-Impaired Subjects (n=10) |
| Albumin (g/dL) | 4.3 ± 0.2 | 3.3 ± 0.6 |
| A₁-AGP (mg/dL) | 77.0 ± 18.8 | 46.6 ± 17.1 |
| Total Protein (g/dL) | 7.2 ± 0.2 | 6.9 ± 0.7 |
| Unbound Fraction (+) R078543 | 0.215 ± 0.0469 | 0.306 ± 0.0687 |
| Unbound Fraction (-) R078544 | 0.385 ± 0.0416 | 0.457 ± 0.0504 |
| Unbound Fraction Paliperidone | 0.279 ± 0.0492 | 0.353 ± 0.0564^{a} |

| | | |
|---|---|---|
| All values are mean (SD). ^{a}Descriptive statistics based on n=8, excluding Subjects 0005 and 0006. | | |

Overall, hepatically-impaired subjects achieved lower total plasma concentrations than healthy subjects. AUC and Cₘₐₓ values of Paliperidone and each of its enantiomers were lower for hepatically impaired subjects than for healthy subjects: in each case, Cₘₐₓ was approximately 35% lower and AUC_{∞} approximately 27% lower. After correction for unbound fraction, the exposure was comparable between the groups. The median time to reach maximum plasma concentration was around 1 hour for both groups, although somewhat more variable among the hepatically-impaired subjects.

Paliperidone plasma concentration declined with a mean terminal half-life of 23.6 hours for healthy subjects and 26.5 hours for hepatically-impaired subjects.

The CL/F for Paliperidone was about 35% higher in hepatically-impaired subjects compared with healthy subjects which is consistent with the lower AUC_{∞}. Moreover, hepatically-impaired subjects had 47% higher volumes of distribution for total Paliperidone compared with healthy subjects. Based on unbound concentrations, however, the clearance and volume of distribution were comparable between the groups.

Hepatically-impaired subjects showed more variable renal excretion profiles (i.e. larger %CV) than healthy subjects. There were no other apparent differences in urinary excretions parameters between the hepatic function groups. Approximately 50% of the dose was excreted unchanged into urine and did not differ between the groups. Renal clearance was not much different between the groups (67.4 vs. 51.2 ng/ml), which can be expected because the unbound plasma concentrations between the goups are comparable. Renal function, as determined by the creatinine clearance, was almost identical between the groups. Active renal clearance accounted on average for approximately 45% of the renal clearance in both groups.

| **Paliperidone** | | | | | | | | |
|---|---|---|---|---|---|---|---|---|
| Healthy Subjects | | | | | Hepatically-Impaired Subjects | | | |
| | **n** | Total | n | **n** Unbound | **n** | Total | **n** | Unbound |
| C_{max,}ng/mL | 10 | 7.14 ± 2.28 | 10 | 1.81 ± 0.292 | 10 | 4.57 ± 1.05 | 10 | 1.59 ± 0.318 |
| AUC_{∞}, ng.h/ml | 10 | 176 ± 64.4 | 10 | 45.8 ± 8.72 | 9 | 128 ± 42.5 | 8 | 45.7 ± 12.6 |
| tₘₐₓ , h | 10 | 1.00 (1.00-2.00) | 10 | 1.25 (1.00-2.00) | 10 | 1.25 (0.25-4.00) | 10 | 1.25 (0.25-4.00) |
| t_{½}, h | 10 | 23.6 ± 3.6 | 10 | ND | 10 | 26.5 ± 6.4 | | ND |
| CL/F, ml/min | 10 | 106 ± 34.9 | 10 | 370 ± 67.1 | 9 | 143 ± 43.4 | 8 | 386 ± 99.3 |
| V_{dZ}, L | 10 | 211 ± 59.6 | 10 | 748 ± 144 | 9 | 31 1 ± 65.2 | 8 | 857 ± 146 |
| CL_{R}, ml/min | 10 | 51.2 ± 13.4 | | ND | 9 | 67.4 ± 34.0 | | ND |
| CL_{NR}, ml/min | 10 | 54.4 ± 23.7 | 10 | 188 ± 56.8 | 9 | 75.1 ± 16.2 | 8 | 205 ± 30.7 |
| Ae,%dose | 10 | 50.1 ± 7.94 | | ND | 10 | 44.7 ± 10.62 | | ND |
| Mean ± SD; for tₘₐₓ median (range); ND: Not determined. | | | | | | | | |

When the data from the two hepatic function groups were pooled, there was no apparent relationship between clearance of paliperidone or its enantiomers and most measures of hepatic function (i.e. albumin and bilirubin concentrations, prothrombin time, and Child-Pugh score); there was an inverse relationship between clearance of paliperidone or its separate enantiomers and α₁-AGP concentration.

Exposure to both enantiomers was higher in healthy subjects compared with hepatically-impaired subjects; furthermore, in both groups, exposure to (+) R078543 was high than exposure to R078544. The (+)/(-) ration based on the AUC for the total plasma concentrations was somewhat larger in healthy subjects compared with hepatically-impaired subjects (i.e. 1.67 and 1.38 respectively). Based on unbound concentrations, however, the exposure to both enantiomers was within the same range, and the (+)/(-) AUC ration was comparable between healthy and hepatically-impaired subject (i.e., 0.914 and 0.886, respectively).

Safety Results: The only adverse events that were reported in more than I subject in either group were hyperproclactinemia (see below) and dizziness (in 2 hepatically-impaired subjects only). Treatment-emergent increase in hepatic enzymes were noted in 1 hepatically-impaired and 1 healthy subject. These elevations were only slightly above the baseline value in he hepatically-impaired subject and less than twice the upper limit of normal in the health subject, and thus were not considered clinically important.

An increase in proclactin from the mean predose levels was seen in both hepatically-impaired and healthy subjects at 36 hours; thereafter mean levels decreased. Because the investigator was unblinded to the laboratory results, increases in prolactin levels were reported as adverse events in 8 hepatically-impaired and 6 healthy subjects; these adverse events were considered mild and very likely related to study drug by the investigator.

There were no unexpected findings in vital signs; no subject in either group met the criteria for orthostatic hypotension. Furthermore, no subjects had clinically important abnormal ECG values (including QT values).

### Conclusion:

After oral administration of 1 mg paliperidone IR, hepatically impaired subject had a lower mean Cₘₐₓ (≈35%) and AUC_{∞} (≈27%) for total paliperidone and its enantiomers than did healthy subjects.

The protein binding differed between the hepatic function groups. The unbound fraction of palieridone was approximately 27% higher in hepatically-impaired subjects. Taking this difference in protein binding into account Cₘₐₓ and AUC_{∞} for the unbound fracon of palpridone were comparable across the hepatic function groups. Cₘₐₓ was approximately 12% lower, and AUC_{∞} approximately 5% lower, in hepatically-impaired subjects compared with healthy subjects

The mean terminal half-life for IR paliperidone and its enantiomers was between 23.6 and 25.0 hours for healthy subjects, and between 26.5 and 27.5 hours for hepatically-impaied subjects.

Paliperione IR, I mg, was tolerated equally well by healthy and hepatically-impaired subjects.

## Claims

1. Paliperidone, its pharmaceutically acceptable acid addition salts, enantiomeric forms and esters thereof for treating psychiatric patients having or at risk of hepatic impairment.

2. Paliperidone, its pharmaceutically acceptable acid addition salts, enantiomeric forms and esters thereof according to claim 1, wherein the psychiatric patient is in need of treatment for psychosis.

3. Paliperidone, its pharmaceutically acceptable acid addition salts, enantiomeric forms and esters thereof according to claim 2, wherein the psychiatric patient is in need of treatment for schizophrenia.

4. Paliperidone, its pharmaceutically acceptable acid addition salts, enantiomeric forms and esters thereof according to claim 2, wherein the psychiatric patient is in need of treatment for bipolar disorder.

5. Paliperidone, its pharmaceutically acceptable acid addition salts, enantiomeric forms and esters thereof according to claim 1, wherein the psychiatric patient is in need of treatment for a mental disorder selected from the group consisting of Mild Mental Retardation (317), Moderate Mental Retardation (318.0), Severe Mental Retardation (318.1), Profound Mental Retardation (318.2), Mental Retardation Severity Unspecified (319), Autistic Disorders (299.00), Rett's Disorder (299.80), Childhood Disintegrative Disorders (299.10), Asperger's Disorder (299.80), Pervasive Developmental Disorder Not Otherwise Specified (299.80), Attention-Deficit/Hyperactivity Disorder Combined Type (314.01), Attention-Deficit/Hyperactivity Disorder Predominately Inattentive Type (314.00), Attention-Deficit/Hyperactivity Disorder Predominately Hyperactive-Impulsive Type (314.01), Attention-Deficit/Hyperactivity Disorder NOS (314.9), Conduct Disorder (Childhood-Onset and Adolescent Type 312.8), Oppositional Defiant Disorder (313.81), Disruptive Behavior Disorder Not Otherwise Specified (3 12.9), Solitary Aggressive Type (312.00), Conduct Disorder, Undifferentiated Type (312.90), Tourette's Disorder (307.23), Chronic Motor Or Vocal Tic Disorder (307.22), Transient Tic Disorder (307.21), Tic Disorder NOS (307.20), Alcohol Intoxication Delirium (291.0), Alcohol Withdrawal Delirium (291.0), Alcohol-Induced Persisting Dementia (291.2), Alcohol-Induced Psychotic Disorder with Delusions (291.5), Alcohol-Induced Psychotic Disorder with Hallucinations (291.3), Amphetamine or Similarly Acting Sympathomimetic Intoxication (292.89), Amphetamine or Similarly Acting Sympathomimetic Delirium (292.81), Amphetamine or Similarly Acting Sympathomimetic Induced Psychotic with Delusional (292.11), Amphetamine or Similarly Acting Sympathomimetic Induced Psychotic with Hallucinations (292.12), Cannabis-Induced Psychotic Disorder with Delusions (292.11), Cannabis-Induced Psychotic Disorder with Hallucinations (292.12), Cocaine Intoxication (292.89), Cocaine Intoxication Delirium (292.81), Cocaine-Induced Psychotic Disorder with Delusions (292.11), Cocaine-Induced Psychotic Disorder with Hallucinations (292.12), Halluciogen Intoxication (292.89), Hallucinogen Intoxication Delirium (292.81), Hallucinogen-Induced Psychotic disorder with Delusions (292.11), Hallucinogen-Induced Psychotic disorder with Delusions (292.12), Hallucinogen-Induced Mood Disorder (292.84), Hallucinogen-Induced Anxiety Disorder (292.89), Hallucinogen-Related Disorder Not Otherwise Specified (292.9), Inhalant Intoxication (292.89), Inhalant Intoxication Delirium (292.81), Inhalant-Induced Persisting Dementia (292.82), Inhalant-Induced Psychotic Disorder with Delusions (292.11), Inhalant-Induced Psychotic with Hallucinations (292.12), Inhalant-Induced Mood Disorder (292.89), Inhalant-Induced Anxiety Disorder (292.89), Inhalant-Related Disorder Not Otherwise Specified (292.9), Opioid Intoxication Delirium (292.81), Opioid-Induced Psychotic Disorder with Delusions (292.11), Opioid Intoxication Delirium (292.81), Opioid-Induced Psychotic Disorder with Hallucinations (292.12), Opioid-Induced Mood Disorder (292.84), Phencyclidine (PCP) or Similarly Acting Arylcyclohexylamine Intoxication (292.89), Phencyclidine (PCP) or Similarly Acting Arylcyclohexylamine Intoxication Delirium (292.81), Phencyclidine (PCP) or Similarly Acting Arylcyclohexylamine Induced Psychotic Disorder with Delusions (292.11), Phencyclidine (PCP) or Similarly Acting Arylcyclohexylamine Induced Psychotic Disorder with Hallucinations (292.12), Phencyclidine (PCP) or Similarly Acting Arylcyclohexylamine Mood Disorder (292.84), Phencyclidine (PCP) or Similarly Acting Arylcyclohexylamine Induced Anxiety Disorder (292.89), Phencyclidine (PCP) or Similarly Acting Arylcyclohexylamine Related Disorder Not Otherwise Specified (292.9), Sedative, Hypnotic or Anxiolytic Intoxication (292.89), Sedation, Hypnotic or Anxiolytic Intoxication Delirium (292.81), Sedation, Hypnotic or Anxiolytic Withdrawal Delirium (292.81), Sedation, Hypnotic or Anxiolytic Induced Persisting Dementia (292.82), Sedation, Hypnotic or Anxiolytic-Induced Psychotic Disorder with Delusions (292.11), Sedation, Hypnotic or Anxiolytic-Induced Psychotic Disorder with Hallucinations (292.12), Sedation, Hypnotic or Anxiolytic-Induced Mood Disorder (292.84), Sedation, Hypnotic or Anxiolytic-Induced Anxiety Disorder (292.89), Other (or Unknown) Substance Intoxication (292.89), Other (or Unknown) Substance-Induced Delirium (292.81), Other (or Unknown) Substance-Induced Persisting Dementia (292.82), Other (or Unknown) Substance-Induced Psychotic Disorder with Delusions (292.11), Other (or Unknown) Substance-Induced Psychotic Disorder with Hallucinations (292.12), Other (or Unknown) Substance-Induced Mood Disorder (292.84), Other (or Unknown) Substance-Induced Anxiety Disorder (292.89), Other (or Unknown) Substance Disorder Not Otherwise Specified (292.9), Obsessive Compulsive Disorder (300.3), Post-traumatic Stress Disorder (309.81), Generalized Anxiety Disorder (300.02), Anxiety Disorder Not Otherwise Specified (300.00), Body Dysmorphic Disorder (300.7), Hypochondriasis (or Hypochondriacal Neurosis) (300.7), Somatization Disorder (300.81), Undifferentiated Somatoform Disorder (300.81), Somatoform Disorder Not Otherwise Specified (300.81), Intermittent Explosive Disorder (312.34), Kleptomania (312.32), Pathological Gambling (312.31), Pyromania (312.33), Trichotillomania (312.39), and Impulse Control Disorder NOS (312.30), Schizophrenia, Paranoid Type, (295.30), Schizophrenia, Disorganized (295.10), Schizophrenia, Catatonic Type, (295.20), Schizophrenia, Undifferentiated Type (295.90), Schizophrenia, Residual Type (295.60), Schizophreniform Disorder (295.40), Schizoaffective Disorder (295.70), Delusional Disorder (297.1), Brief Psychotic Disorder (298.8), Shared Psychotic Disorder (297.3), Psychotic Disorder Due to a General Medical Condition with Delusions (293.81), Psychotic Disorder Due to a General Medical Condition with Hallucinations (293.82), Psychotic Disorders Not Otherwise Specified (298.9), Major Depression, Single Episode, Severe, without Psychotic Features (296.23), Major Depression, Recurrent, Severe, without Psychotic Features (296.33), Bipolar Disorder, Mixed, Severe, without Psychotic Features (296.63), Bipolar Disorder, Mixed, Severe, with Psychotic Features (296.64), Bipolar Disorder, Manic, Severe, without Psychotic Features (296.43), Bipolar Disorder, Manic, Severe, with Psychotic Features (296.44), Bipolar Disorder, Depressed, Severe, without Psychotic Features (296.53), Bipolar Disorder, Depressed, Severe, with Psychotic Features (296.54), Bipolar II Disorder (296.89), Bipolar Disorder Not Otherwise Specified (296.80), Personality Disorders, Paranoid (301.0), Personality Disorders, Schizoid (301.20), Personality Disorders, Schizotypal (301.22), Personality Disorders, Antisocial (301.7), and Personality Disorders, Borderline (301.83).

6. Paliperidone, its pharmaceutically acceptable acid addition salts, enantiomeric forms and esters thereof according to any preceding claim, wherein the paliperidone ester is paliperidone palmitate.
